# EUROPÄISCHE PATENTSCHRIFT

(11) **EP 0 679 635 B1**
(45) Veröffentlichungstag und Bekanntmachung des Hinweises auf die Patenterteilung: **27.09.2000**
(21) Anmeldenummer: 95105726.4
(22) Anmeldetag: 18.04.1995
(51) Int. Cl.: C07C 245/20, C07C 323/48, C07D 209/88, G03F 7/021

(54) **Aromatische Diazoniumsalze und deren Verwendung in strahlungsempfindlichen Gemischen**
Aromatic diazonium salts and their use in radiation-sensitive compositions
Les sels diazonium aromatiques et leur utilisation dans les compositions photosensibles

(30) Priorität: 28.04.1994 DE 4414897
(43) Veröffentlichungstag der Anmeldung: 02.11.1995
(73) Patentinhaber: AGFA-GEVAERT N.V., 2640 Mortsel (BE)
(72) Erfinder: Eichhorn, Mathias, Dr., D-65527 Niedernhausen (DE); Buhr, Gerhard, Dr., D-61462 Königstein (DE)

(56) Entgegenhaltungen:
- EP-A- 0 102 450
- EP-A- 0 353 600
- EP-A- 0 530 815
- DE-A- 2 024 243
- DE-A- 2 641 099

## Beschreibung

Die Erfindung betrifft aromatische Diazoniumverbindungen und ein Verfahren zu ihrer Herstellung sowie ein strahlungsempfindliches Gemisch, das
a) eine Verbindung, die unter der Einwirkung aktinischer Strahlung Säure bildet,
b¹) für ein positiv-arbeitendes Gemisch eine Verbindung, deren Löslichkeit in wäßrig-alkalischer Lösung unter der Einwirkung der Strahlung oder der Säure zunimmt, oder
b²) für ein negativ-arbeitendes Gemisch eine Verbindung, deren Löslichkeit in wäßrig-alkalischer Lösung unter der Einwirkung dieser Säure abnimmt, und gegebenenfalls
c) ein wasserunlösliches, in wäßrig-alkalischer Lösung dagegen lösliches oder zumindest quellbares polymeres organisches Bindemittel
enthält. Darüber hinaus betrifft die Erfindung ein Aufzeichnungsmaterial mit einem Träger und einer strahlungsempfindlichen Schicht.

Aromatische Diazoniumsalze sind seit langem bekannt. So ist in der DE-A 20 24 242 (= US-A 3 849 392) eine Reihe von Diazoniumsalzen beschrieben. Konkret offenbart sind insbesondere 4-Anilino-benzoldiazonium-, 4-Anilino-2-methoxy-benzoldiazonium-, 4-(4-Methoxy-anilino)-benzoldiazonium-, 4-(2-Carboxy-anilino)-benzoldiazonium-, 2,5-Diethoxy-4-(4-ethoxy-anilino)-benzoldiazonium-, 2,5-Dimethoxy-4-p-tolylmercapto-benzoldiazonium-, 2,5-Dimethoxy-4-phenoxy-benzoldiazonium-, 4-(2,5-Diethoxy-benzoylamino)-2,5-diethoxy-benzoldiazonium-, 3-Methoxy-dibenzofuran-2-diazonium-, 4-(Methyl-naphthalin-1-ylmethyl-amino)-benzoldiazonium-, 4-Anilino-2-carboxy-benzoldiazonium, 2,5-Dimethoxy-4-(N-methyl-N-phenylmercaptoacetylamino)-benzoldiazonium- und 4-[N-Methyl-N-(2-phenylmercapto-ethyl)-amino]-benzoldiazoniumsalz. Alle diese Salze liegen in Form der Chloride, Sulfate oder Phosphate vor. Kondensationsprodukte dieser Salze wurden in strahlungsempfindlichen Gemischen verwendet.

In der DE-A 26 41 099 (= US-A 4 163 672) sind lichtempfindliche, bei Bestrahlung Säure abspaltende 2,4,5-trisubstituierte Benzoldiazoniumsalze beschrieben. Diese Salze liegen als Hexafluorphosphate, Tetrafluorborate oder Hexafluorarsenate vor. Sie wurden in einem lichtempfindlichen Gemisch verwendet, das neben einem dieser Salze Ester oder Amide einer ortho-Naphthochinondiazidsulfonsäure oder -carbonsäure und einen zur Salzbildung befähigten Triphenylmethan-, Azin- oder Anthrachinonfarbstoff enthält.

Die sogenannten "chemisch verstärkten" Gemische sind besonders lichtempfindlich. Sie enthalten im allgemeinen eine Verbindung, die unter der Einwirkung von Strahlung ein katalytisch wirksames Agens (gewöhnlich eine Säure) freisetzt, und eine Verbindung b), die durch die katalytische Wirkung dieses Agens so verändert wird, daß ihre Löslichkeit in einem Entwickler zu- oder abnimmt. In positiv-arbeitenden Gemischen wird diese letztgenannte Verbindung in leichter lösliche Spaltprodukte überführt, während sie in negativ-arbeitenden Gemischen normalerweise in schwerer lösliche, vernetzte Produkte umgewandelt wird. Es sind bereits zahlreiche Verbindungen bekannt, die bei Bestrahlung katalytisch wirksame Säure freisetzen. Zu diesen "Photostartern" gehören Triazine, die mit Trihalogenmethylgruppen substituiert sind. Bei Bestrahlung setzen sie Halogenwasserstoffsäure frei. Halogenwasserstoffsäuren sind jedoch relativ flüchtig, so daß sie häufig aus der Kopierschicht heraus diffundieren und dadurch nicht die volle katalytische Wirksamkeit entfalten. Die starke Nucleophilie des Halogenid-Anions führt außerdem dazu, daß ein erheblicher Teil der Säure durch unerwünschte Nebenreaktionen verbraucht wird.

In der EP-A 0 102 450 ist ein chemisch verstärktes, lichtempfindliches Gemisch beschrieben, in dem als Photostarter Aryldiazonium-, Triarylsulfonium- oder Diaryliodoniumsalze verwendet werden. Diese Salze liegen als Tetrafluorborate, Hexafluorantimonate oder Hexafluorphosphate vor. Die aus diesen Salzen bei Bestrahlung freigesetzten Säuren sind weniger flüchtig als Halogenwasserstoffsäuren, und das dazugehörige Anion ist auch weniger nucleophil. Nachteilig ist jedoch der Metallanteil. Wird ein Halbleitersubstrat mit einem Gemisch beschichtet, das ein derartiges Salz enthält, so kann es zu einer unerwünschten Dotierung des Substrats mit diesen Metallen kommen.

In der EP-A 0 353 600 sind aromatische Diazoniumsalze beschrieben, in deren Anion keine solchen Metalle vorkommen. Diese Salze liegen als Methansulfonate, Trifluormethansulfonate oder Trifluoracetate vor. Diese Salze sind jedoch in vielen der üblicherweise für lichtempfindliche Gemische verwendeten Lösemitteln nicht ausreichend löslich. Ein weiterer Nachteil ist die hohe Kristallisationsneigung dieser Salze.

Es bestand daher die Aufgabe, Diazoniumsalze bereitzustellen, die als Photostarter geeignet sind, ohne dabei die beschriebenen Nachteile aufzuweisen. Sie sollen außerdem möglichst wenig toxisch sein.

Gelöst wird die Aufgabe durch aromatische und heteroaromatische Mono- oder Bis-diazonium-1,1,2,3,3,3-hexafluorpropansulfonate.

Gegenstand der vorliegenden Anmeldung sind damit als Photostarter in strahlungsempfindlichen Gemischen geeignete aromatische und heteroaromatische Mono- oder Bis-diazonium-1,1,2,3,3,3-hexafluorpropansulfonate, in denen das aromatische oder heteroaromatische Kation 4 bis 20 Kohlenstoffatome enthält, wobei die Heteroatome in den heteroaromatischen Verbindungen Sauerstoff-, Schwefel- und/oder Stickstoffatome sind.

Von den aromatischen Diazonium-1,1,2,3,3,3-Hexafluorpropansulfonaten sind gegebenenfalls substituierte Benzoldiazonium-1,1,2,3,3,3-hexafluorpropansulfonate und Fluorendiazonium-1,1,2,3,3,3-hexafluorpropansulfonate bevorzugt. Die gegebenenfalls vorhandenen Substituenten sind bevorzugt Halogenatome, Hydroxy-, (C₁-C₄)Alkoxy-, Phenoxy-, (C₁-C₄)Alkyl-, Amino-,(C₁-C₄)Alkylamino-, Di(C₁-C₄)alkylamino-, (C₁-C₄)Hydroxyalkylamino-, Di (C₁-C₄)hydroxyalkylamino-, (C₁-C₄)Hydroxyalkyl-(C₁-C₄)alkyl-amino-, Pyrrolidino-, Piperidino-, Morpholino-, Phenylamino-, Nitro-, Phenyl- oder Phenylazogruppen. Sie können auch Benzoylgruppen sein, die gegebenenfalls noch mit einer (C₁-C₄)Alkyl-, (C₁-C₄)Alkoxy- oder Diazoniumgruppe substituiert sind. Schließlich können die gegebenenfalls vorhandenen Substituenten auch Phenylmercaptogruppen sein, die ebenso gegebenenfalls noch mit einer (C1-C4)Alkyl- oder (C1-C4)Alkoxygruppe substituiert sind.

Von den heteroaromatischen Diazonium-1,1,2,3,3,3-hexafluorpropansulfonaten sind Dibenzofuran-x-diazonium-1,1,2,3,3,3-hexafluorpropansulfonate, Dibenzo[b,d]thiophen-x-diazonium-1,1,2,3,3,3-hexafluorpropansulfonate, 9-(C1-C4)Alkyl-carbazol-x-diazonium-1,1,2,3,3,3-hexafluorpropansulfonate bevorzugt, wobei x die Positionsziffer angibt. Die Diazoniumgruppe kann allgemein an jeder der Positionen 1 bis 8 der genannten Heterocyclen stehen.

Die Herstellung der erfindungsgemäßen Diazoniumsalze der 1,1,2,3,3,3-Hexafluorpropansulfonsäure (= HFPSA) kann nach Verfahren erfolgen, die dem Fachmann an sich bekannt sind. Zum Beispiel können sie aus den entsprechenden (hetero)aromatischen Diazoniumhalogeniden oder -sulfaten durch Umsetzung mit HFPSA (Methode A) oder durch Diazotierung eines (hetero)aromatischen Amins in Gegenwart von HFPSA (Methode B) hergestellt werden.

Bei der Methode A wird allgemein das in Lösung befindliche Diazoniumhalogenid oder -sulfat unter Rühren mit HFPSA versetzt. Das Lösemittel wird dabei zweckmäßig so gewählt, daß das dabei gebildete Diazonium-1,1,2,3,3,3-hexafluorpropansulfonat als Feststoff ausfällt und abgetrennt werden kann. Ein Lösemittel, das diese Anforderungen in vielen Fällen erfüllt, ist Wasser. In anderen Fällen können organische Lösemittel, wie Ether, Ketone oder Alkohole, vorteilhafter sein.

Die Diazotierung aromatischer Amine in Gegenwart starker Säuren ist seit langem bekannt. Normalerweise erfolgt sie in wäßriger Lösung unter Verwendung von Natriumnitrit, wobei die entstehenden Diazoniumsalze zumindest teilweise gelöst bleiben. Bei der Darstellung der erfindungsgemäßen Diazoniumsalze werden die entsprechenden (Hetero)arylamine vorteilhaft in organischen Lösemitteln mit Isoamylnitrit diazotiert. Die Diazonium-1,1,2,3,3,3-hexafluorpropansulfonate fallen dabei in feinteiliger, gut zu isolierender Form an. Nur ausnahmsweise muß vorher noch ein Fällungsmittel zugesetzt werden.

Das erfindungsgemäße strahlungsempfindliche Gemisch, das
a) eine Verbindung, die unter der Einwirkung aktinischer Strahlung Säure bildet,
b¹) für ein positiv-arbeitendes Gemisch eine Verbindung, deren Löslichkeit in wäßrig-alkalischer Lösung unter der Einwirkung der Strahlung oder der Säure zunimmt, oder
b²) für ein negativ-arbeitendes Gemisch eine Verbindung, deren Löslichkeit in wäßrig-alkalischer Lösung unter der Einwirkung der Säure abnimmt, und gegebenenfalls
c) ein wasserunlösliches, in wäßrig-alkalischer Lösung dagegen lösliches oder zumindest quellbares polymeres organisches Bindemittel
enthält, ist dadurch gekennzeichnet, daß die Verbindung a) ein aromatisches oder heteroaromatisches Diazoniumsalz der 1,1,2,3,3,3-Hexafluorpropansulfonsäure ist. Bevorzugte aromatische oder heteroaromatische Diazoniumsalze sind oben angegeben.

Die Verbindung b¹), deren Löslichkeit in wäßrig-alkalischer Lösung unter der Wirkung der Säure zunimmt, ist vorzugsweise ein Polymer mit säurelabilen Gruppen. Bevorzugt sind dabei Polymere, die mindestens eine durch Säure spaltbare C-O-C-Bindung aufweisen. Hierzu zählen Polymere, in denen aromatische Ringe mit säurespaltbaren tert.-Butoxycarbonyloxy-, tert.-Butoxycarbonyl- oder tert.-Butoxygruppen substituiert sind. Besonders bevorzugte Verbindungen b¹) sind gegebenenfalls substituierte, insbesondere alkylsubstituierte, Polyhydroxystyrole, deren phenolische Hydroxygruppen vollständig oder teilweise durch tert.-Butoxycarbonyloxy-, tert.-Butoxycarbonyl-oder tert.-Butoxygruppen ersetzt sind.

Solche Polymere erhält man zum Beispiel aus Polyhydroxystyrolen oder Novolaken durch teilweise oder vollständige Umsetzung mit Di-tert.-butyl-dicarbonat. Besonders geeignet sind auch Polymere mit Gruppen aus tert.-Butylmethacrylat. Weiterhin zählen dazu Acetale und Ketale mit säurespaltbaren C-O-C-Bindungen. Geeignete niedermolekulare Acetale und Ketale sind in der DE-A 26 10 842 (= US-A 4 101 323), geeignete polymere Acetale und Ketale in der DE-A 27 18 254 (= US-A 4 247 611) beschrieben. Geeignet sind weiterhin auch Poly(N,O-acetale) wie sie in der EP-A 0 510 449 (= US-A 5 286 602) beschrieben sind. Der Anteil dieser säurelabilen Polymere beträgt etwa 60 bis 99 Gew.-%, bezogen auf das Gesamtgewicht der nichtflüchtigen Bestandteile des Gemisches. Die Menge bezieht sich auf ein Gemisch ohne eines der polymeren Bindemittel c). Bei Verwendung der genannten polymeren säurespaltbaren Verbindungen ist das polymere Bindemittel c) nicht unbedingt erforderlich. Wird jedoch eines dieser Bindemittel verwendet, verringert sich der Anteil der säurelabilen Polymere entsprechend.

Die Verbindung b¹), deren Löslichkeit in wäßrig-alkalischer Lösung unter der Einwirkung der aktinischen Strahlung zunimmt, ist vorzugsweise ein ortho-Chinondiazid, besonders bevorzugt ein ortho-Naphthochinondiazid. Besonders geeignete ortho-Naphthochinondiazide sind gegebenenfalls noch weiter substituierte Bis-[4-(1,2-naphthochinon-2-diazid-4- oder -5-sulfonyloxy)-phenyl]-(C₂-C₉)alkansäuren und deren Ester, 2,3,4,2',3',4'-Hexakis-(1,2-naphthochinon-2-diazid-4- oder - 5-sulfonyloxy)-5,5'-dialkanoyl- oder -diaroyl-diphenylmethane und gegebenenfalls noch weiter substituierte Bis-[4,4'-(1,2-naphthochinon-2-diazid-4- oder -5-sulfonyloxy)-benzoyl]-(C₂-C₁₈)alkane. Allgemein sind Ester aus 1,2-Naphthochinon-2-diazid-4- oder -5-sulfonsäure und Verbindungen mit phenolischen Hydroxygruppen, insbesondere solchen mit 2 bis 6 phenolischen Hydroxygruppen, geeignet. Die erfindungsgemäßen Diazoniumsalze haben in solchen Gemischen die Funktion eines Kontrastbildners. Der besondere Vorteil liegt darin, daß die thermische Stabilität von latenten Bildern aus Aufzeichnungsmaterialien, die mit diesem Gemisch hergestellt sind, deutlich erhöht ist. Als Vergleich wurden Aufzeichnungsmaterialien herangezogen, deren strahlungsempfindliche Schicht andere strahlungsempfindliche Kontrastbildner, wie 1,2-Naphthochinon-2-diazid-4-sulfonylchlorid oder substituierte Trihalogenmethyl-s-triazine, enthält.

Die Verbindung b²), deren Löslichkeit in wäßrig-alkalischer Lösung unter der Einwirkung der Säure abnimmt, enthält vorzugsweise mindestens zwei durch Säure vernetzbare Gruppen, insbesondere mindestens zwei Hydroxymethyl-, Methoxymethyl- oder Acetoxymethylgruppen. Dazu zählen Verbindungen der Formeln (R¹O-CHR³)ₙ-A-(CHR³-OR²)ₘ und (R¹O-CHR³)ₙ-A-Y-A-(CHR³-OR²)ₘ, worin
- A: einen gegebenenfalls substituierten einkernigen aromatischen Kohlenwasserstoff oder eine Sauerstoff, Schwefel oder Stickstoff enthaltende heterocyclische aromatische Verbindung,
- Y: eine Einfachbindung, (C₁-C₄)Alkylen oder (C₁-C₄)Alkylendioxy, wobei Kohlenstoffketten durch Sauerstoffatome unterbrochen sein können, -O-, -S-, -SO₂-, -CO-, -CO-O-, -O-CO-O-, -CO-NH- oder Phenylendioxy,
- R¹ und R²: gleich oder verschieden sind und Wasserstoffatome, (C₁-C₄)Alkyl-, Cycloalkyl-, gegebenenfalls substituierte Aryl-, Aralkyl- oder Alkanoylgruppen,
- R³: ein Wasserstoffatom, eine (C₁-C₄)Alkyl- oder eine gegebenenfalls substituierte Phenylgruppe,
- n: eine ganze Zahl von 1 bis 3 und
- m: eine ganze Zahl von 0 bis 3, mit der Maßgabe, daß n + m mindestes 2 ist,
bedeuten. Auch Hexa-N-methoxymethyl-melamin, nicht gehärtete alkylveretherte Melamin-Formaldehyd-Harze mit einem mittleren Molekulargewicht M_{w} von 500 bis 1.500 (EP-A 0 263 434) und Harnstoff/Formaldehyd- oder Urethan/Formaldehyd-Kondensationsprodukte (EP-A 0 285 013) sind als Vernetzer geeignet.

Der Anteil der erfindungsgemäßen Diazoniumsalze beträgt allgemein 1 bis 40 Gew.-%, bevorzugt 2 bis 25 Gew.-%, jeweils bezogen auf das Gesamtgewicht der nichtflüchtigen Bestandteile des Gemisches. Enthält das Gemisch eine der obengenannten Verbindungen, deren Löslichkeit in wäßrig-alkalischen Entwicklern unter dem Einfluß von Strahlung zunimmt, so ist der Anteil der erfindungsgemäßen Diazoniumsalze etwas geringer. Er beträgt dann bevorzugt 0,5 bis 20 Gew.-%, besonders bevorzugt 1 bis 10 Gew.-%, wiederum bezogen auf das Gesamtgewicht der nichtflüchtigen Bestandteile des Gemisches.

Ist eine der säurespaltbaren (b¹) oder säurevernetzbaren Verbindungen (b²) niedermolekular, dann enthält das Gemisch zweckmäßig noch ein polymeres Bindemittel c). Geeignet sind Polymere mit phenolischen Hydroxygruppen oder mit Carboxygruppen. Hierzu zählen Phenolharze, insbesondere Poly(4-hydroxy-styrol), Cresol/Formaldehyd-Novolake (Schmelzbereich 105 bis 120 °C nach DIN 53 181) und Phenol/Formaldehyd-Novolake (Schmelzbereich 110 bis 120 °C nach DIN 53 181). Auch Copolymere aus Methacrylsäure/ Methylmethacrylat, Crotonsäure/Vinylacetat und Maleinsäureanhydrid/Styrol sind verwendbar. Im Falle der Maleinsäureanhydrid/Styrol-Copolymere bilden sich die Carboxygruppen erst durch alkalische Verseifung der Anhydridgruppen. Das Gemisch kann verschiedene Bindemittel gleichzeitig enthalten.

Der Anteil des Bindemittels c) beträgt allgemein 30 bis 90 Gew.-%, bevorzugt 55 bis 85 Gew.-%, jeweils bezogen auf das Gesamtgewicht der nichtflüchtigen Bestandteile des Gemisches.

Neben den genannten Bestandteilen kann das erfindungsgemäße Gemisch auch noch bis zu etwa 20 Gew.-%, bezogen auf das Gesamtgewicht der nichtflüchtigen Bestandteile des Gemisches, an anderen Homo- oder Copolymeren enthalten. Dies sind insbesondere Vinylpolymere, wie Polyvinylacetate, Polyacrylate, Polyvinylether, die durch den Einbau von Comonomeren modifiziert sein können. Mit diesen zusätzlichen Polymeren lassen sich die Entwicklungseigenschaften beeinflussen.

Um bestimmte Eigenschaften, wie Flexibilität, Haftung, Glanz, UV-Absorption etc., zu beeinflussen, kann das erfindungsgemäße Gemisch - je nach Anwendungszweck - schließlich noch untergeordnete Mengen an Polyglykolen, Cellulosederivaten, wie Ethylcellulose, Netzmitteln, UV-Absorbern, Farbstoffen und feinteiligen Pigmenten enthalten. Als Farbstoffe haben sich die Triphenylmethanfarbstoffe, insbesondere in Form ihrer Carbinolbasen, bewährt.

Gegenstand der vorliegenden Erfindung ist schließlich auch ein Aufzeichnungsmaterial mit einem Träger und einer strahlungsempfindlichen Schicht, das dadurch gekennzeichnet ist, daß die Schicht aus dem erfindungsgemäßen strahlungsempfindlichen Gemisch besteht.

Das Aufzeichnungsmaterial wird hergestellt, indem eine Lösung des erfindungsgemäßen Gemisches auf den Träger aufgebracht und das Lösemittel anschließend entfernt wird. Als Lösemittel geeignet sind Ketone (wie Butanon), chlorierte Kohlenwasserstoffe (Trichlorethylen und 1,1,1-Trichlorethan), Alkohole (wie Propanol), Ether (wie Tetrahydrofuran), Glykol-monoalkylether (wie Ethylenglykol-monoethylether und Propylenglykol-monomethylether), Glykol-monoalkylether-acetate (wie Propylenglykol-monomethylether-acetat) und Ester (wie Butylacetat). Es können auch Gemische davon verwendet werden. Als zusätzliches Lösemittel kann noch Acetonitril, Dioxan oder Dimethylformamid vorhanden sein. Allgemein sind alle Lösemittel verwendbar, die mit den Bestandteilen des erfindungsgemäßen Gemisches oder dem Trägermaterial nicht in unerwünschter Weise reagieren.

Lösemittel und Feststoffanteil in der Lösung richten sich auch nach dem Beschichtungs- und Trocknungsverfahren. Schichten bis etwa 5 µm Dicke werden im allgemeinen aufgeschleudert oder mit einem Streichrakel aufgetragen und anschließend getrocknet. Die dabei verwendeten Lösungen haben einen Feststoffanteil bis zu etwa 40 Gew.-%. Soll das Trägermaterial beidseitig beschichtet werden, so wird es in eine entsprechende Lösung eingetaucht. Niedrig siedende und damit leicht zu entfernende Lösemittel lassen die Schicht schnell antrocknen. Die Beschichtung kann auch durch Aufsprühen oder durch Antragen mit Hilfe von Walzen oder Breitschlitzdüsen erfolgen. Einzelplatten, insbesondere solche aus Zink oder Mehrmetall, werden vorteilhaft durch Gießantrag (curtain coating) beschichtet.

Zur Herstellung von Offsetdruckplatten haben die Aufzeichnungsmaterialien meist einen Träger aus mechanisch und/oder elektrochemisch aufgerauhtem und gegebenenfalls anodisiertem Aluminium, das noch chemisch, z. B. mit Polyvinylphosphonsäure, Silikaten oder Phosphaten, vorbehandelt sein kann. Geeignet sind auch Mehrmetallplatten mit einer Cu/Cr- oder Messing/Cr-Oberfläche. Die darauf aufgebrachten Aufzeichnungsschichten sind gewöhnlich dünner als 10 µm.

Schichten mit einer Dicke von mehr als 10 µm werden gewöhnlich erst auf einen Zwischenträger (normalerweise eine Kunststoffolie) aufgetragen und von dort auf den endgültigen Träger übertragen. Die Kunststoffolie ist häufig eine Polyester- (z. B. Polyethylenterephthalat) oder eine Polyolefinfolie (z. B. Polypropylen).

Zur Herstellung von Hochdruckplatten werden als Trägermaterialien Zink- oder Magnesiumträger sowie deren handelsübliche mikrokristalline Legierungen für Einstufenätzverfahren oder ätzbare Kunststoffe wie Polyoxymethylen verwendet.

Für Tiefdruck- oder Siebdruckformen eignen sich Träger mit Kupfer- oder Nickeloberflächen, auf denen das erfindungsgemäße Gemisch besonders gut haftet. Diese Schichten zeichnen sich auch hier durch die gute Atzfestigkeit aus.

Weiterhin lassen sich die erfindungsgemäßen Gemische als Photoresists und beim Formteilätzen verwenden. Ebenso eignen sie sich zur Beschichtung von
- Leiterplatten aus einem elektrisch nicht-leitenden Material, die ein- oder beidseitig mit einer Kupferauflage versehen sind,
- gegebenenfalls haftvermittelnd vorbehandelten Glas- oder Keramikmaterialien,
- Siliciumscheiben, auf deren Oberfläche sich gegebenenfalls eine Nitrid- oder Oxidschicht befindet; die Beschichtung kann direkt oder mit Hilfe eines Zwischenträgers erfolgen;
- Holz,
- Textilien und den
- Oberflächen vieler Werkstoffe, die durch Projektion bebildert werden und resistent gegen die Einwirkung alkalischer Entwickler sind.

Für die Trocknung der Schicht können die üblichen Geräte und Bedingungen übernommen werden, Temperaturen um 100 °C und kurzfristig bis 120 °C werden ohne Einbuße an Strahlungsempfindlichkeit vertragen.

Zum Belichten können die üblichen Kopiergeräte wie Röhrenlampen, Xenonimpulslampen, metallhalogeniddotierte Quecksilberdampf-Hochdrucklampen und Kohlebogenlampen verwendet werden. Darüber hinaus ist das Belichten in üblichen Projektions- und Vergrößerungsgeräten unter dem Licht der Metallfadenlampen und Kontaktbelichtung mit gewöhnlichen Glühbirnen möglich. Die Belichtung kann auch mit dem kohärenten Licht eines Lasers erfolgen. Geeignet sind leistungsgerechte kurzwellige Laser, beispielsweise Argon-Ionen-Laser, Krypton-Ionen-Laser, Farbstoff-Laser, Helium-Cadmium-Laser sowie Excimer-Laser, die zwischen 193 und 633 nm emittieren. Der Laserstrahl wird mit einer vorgegebenen programmierten Strich- und/oder Rasterbewegung gesteuert.

Im Fall der erfindungsgemäßen, positiv-arbeitenden, strahlungsempfindlichen Gemische kann die bildmäßig belichtete oder bestrahlte Schicht - gegebenenfalls nach einer thermischen Nachbehandlung - mit praktisch den gleichen Entwicklern wie für handelsübliche Naphthochinondiazidschichten und Kopierlacke entfernt werden. Im Fall der erfindungsgemäßen, negativ-arbeitenden, strahlungsempfindlichen Gemische werden durch den Entwickler dagegen die unbelichteten Bereiche entfernt. Die neuen Schichten können in ihren Kopierbedingungen vorteilhaft auf die bekannten Hilfsmittel wie Entwickler und programmierte Sprühentwicklungsgeräte abgestimmt werden. Die wäßrigen Entwicklerlösungen können z. B. Alkaliphosphate, -silikate oder -hydroxide und ferner Netzmittel sowie kleinere Anteile organischer Lösemittel enthalten. In bestimmten Fällen sind auch Lösemittel/Wasser-Gemische als Entwickler brauchbar. Der günstigste Entwickler kann durch Versuche mit der jeweils verwendeten Schicht ermittelt werden. Bei Bedarf kann die Entwicklung mechanisch unterstützt werden. Zur Erhöhung der Widerstandsfähigkeit beim Druck sowie der Resistenz gegen Auswaschmittel, Korrekturmittel und durch UV-Licht härtbare Druckfarben können die entwickelten Platten kurze Zeit auf erhöhte Temperaturen erwärmt werden, wie es für Diazoschichten aus der GB-A 1 154 749 bekannt ist.

Die erfindungsgemäßen Diazonium-1,1,2,3,3,3-hexafluorpropansulfonate können nicht nur in den beschriebenen strahlungsempfindlichen Gemischen eingesetzt werden, sondern überall dort, wo bisher lichtempfindliche Diazoniumsalze benutzt wurden, z. B. zur Herstellung der in der Diazotypie verwendeten Mikrofilme und Lichtpauspapiere.

Im folgenden werden Beispiele für die bevorzugten erfindungsgemäßen Diazonium-1,1,2,3,3,3-hexafluorpropansulfonate und für ihre Anwendung in strahlungsempfindlichen Gemischen gegeben, ohne die Erfindung auf diese zu beschränken.

### Beispiel 1

### Synthese von 2,5-Diethoxy-4-p-tolylmercapto-benzoldiazonium-1,1,2,3,3,3-hexafluorpropansulfonat (Methode A)

50 g 2,5-Diethoxy-4-p-tolylmercapto-benzoldiazoniumchlorid 1/2 ZnCl₂ wurden in 1,5 l Wasser gelöst. Zu der filtrierten Lösung wurden dann unter starkem Rühren tropfenweise 20 ml 1,1,2,3,3,3-Hexafluorpropansulfonsäure gegeben. Das sich in öligen Tropfen abscheidende Produkt erstarrte nach wenigen Minuten, wurde abgesaugt, unter starkem Rühren in 1 l Wasser suspendiert, erneut abgesaugt und schließlich getrocknet. Man erhielt 56,5 g 2,5-Diethoxy-4-p-tolylmercapto-benzoldiazonium-1,1,2,3,3,3-hexafluorpropansulfonat als feines, hellbraunes Pulver.

### Beispiel 2

### Synthese von 9-Ethyl-carbazol-3-diazonium-1,1,2,3,3,3,-hexafluorpropansulfonat (Methode B)

Zu einer Lösung aus 20 ml Tetrahydrofuran (THF) und 4,2 g 9-Ethyl-carbazol-3-ylamin wurden 3 ml 1,1,2,3,3,3-Hexafluorpropansulfonsäure gegeben. Nach Abkühlung auf 0 °C wurden unter Rühren 3 ml iso-Amylnitrit so zugetropft, daß die Temperatur nicht über 10 °C stieg. Nach beendetem Zutropfen wurde das Eisbad entfernt und das Reaktionsgemisch noch 4 h nachgerührt. Es wurden 50 ml Diethylether zugegeben, der entstandene Niederschlag wurde abgenutscht, mit wenig Diethylether gewaschen und getrocknet. Man erhielt 7,4 g 9-Ethyl-carbazol-3-diazonium-1,1,2,3,3,3-hexafluorpropansulfonat als gelbes, feinkristallines Pulver.

### Beispiele 3 bis 9

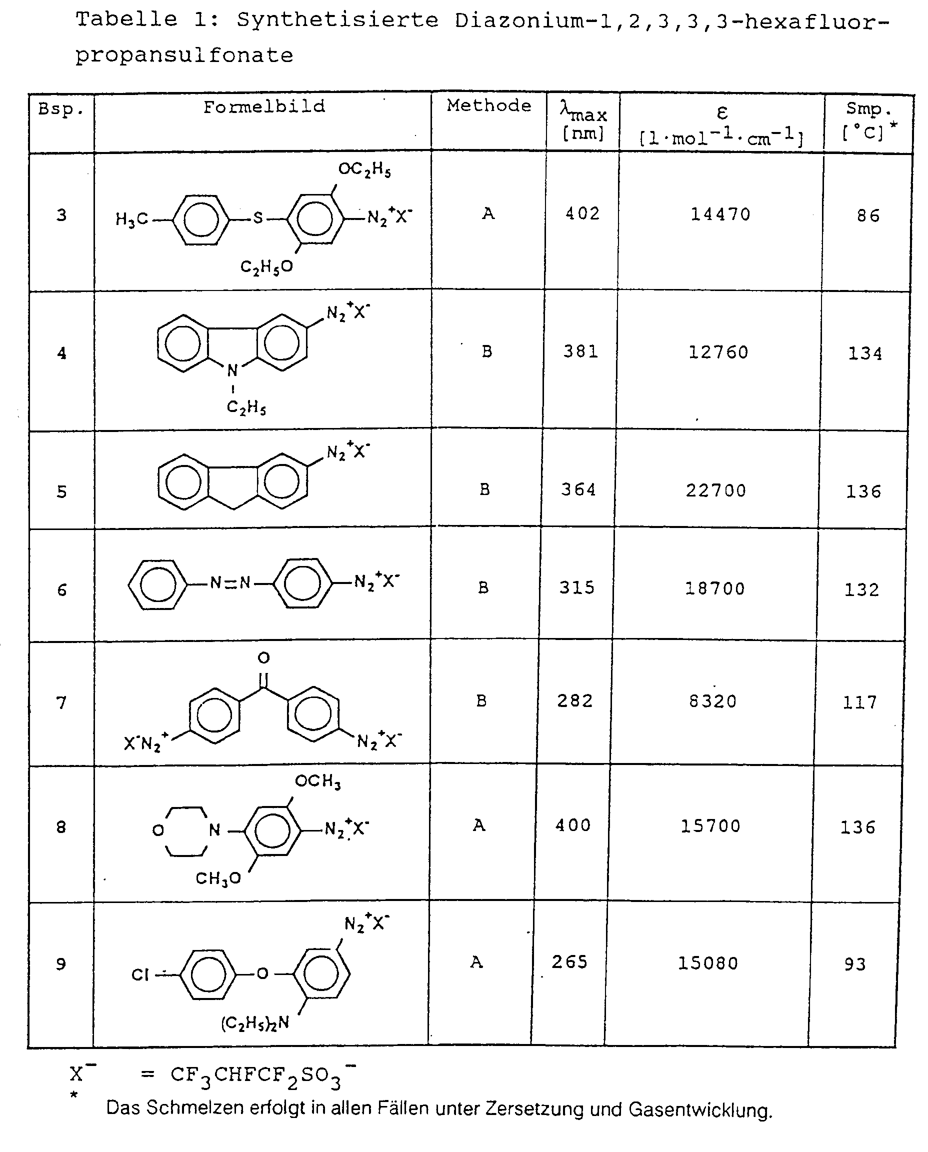

### Beispiele 10 bis 19

Die nachfolgenden Beispiele zeigen die verbesserte Löslichkeit der erfindungsgemäßen Diazonium-1,1,2,3,3,3-hexafluorpropansulfonate in den zur Beschichtung von strahlungsempfindlichen Gemischen vorzugsweise verwendeten Lösemitteln.

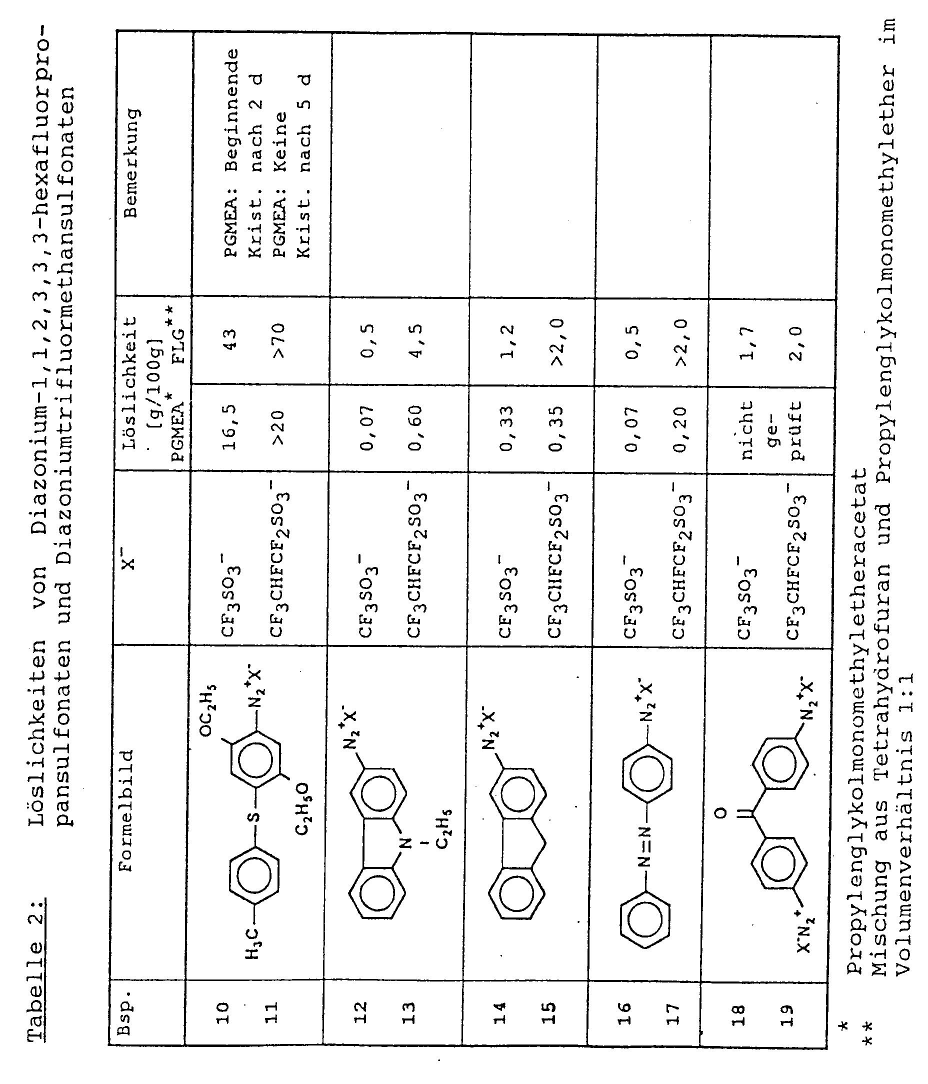

### Beispiele 20 bis 23

Eine Platte aus elektrochemisch aufgerauhtem und anodisiertem Aluminium wurde mit einer Lösung aus
- 9,00 Gt: eines Umsetzungsprodukts aus Polyhydroxystyrol und Di-tert.-butyldicarbonat (OH-Zahl 267),
- 0,50 Gt: Diazonium-1,1,2,3,3,3-hexafluorpropansulfonat gemäß Tabelle 3,
- 0,08 Gt: Kristallviolettbase und
- 175 Gt: Butanon (Methylethylketon)
schleuderbeschichtet und bei 100 °C im Trockenschrank erhitzt. Nach dem Trocknen betrug die Schichtdicke 1,9 µm. Die Platte wurde unter einer 5kW-Metallhalogenidlampe im Abstand von 110 cm durch einen Halbtonstufenkeil mit 13 Stufen einer Dichteabstufung von 0,15 belichtet, 1 min bei 100 °C nacherwärmt und in einem wäßrig-alkalischen Entwickler der Zusammensetzung
- 5,5 Gt: Natriummetasilikat · 9 H₂O,
- 3,4 Gt: Trinatriumphosphat · 12 H₂O,
- 0,4 Gt: Mononatriumphosphat wasserfrei und
- 90,7 Gt: vollentsalztes Wasser
30 s lang entwickelt. Es wurde jeweils ein positives Abbild der Druckvorlage erhalten. Tabelle 3 zeigt, bei welcher Belichtungsdauer die Stufe 3 des Halbtonkeils auf der Platte vollständig offen wiedergegeben wurde.

**Tabelle 3**

| Beispiel | Säurespender gemäß Tab. 1 | Belichtungsdauer in s |
|---|---|---|
| 20 | 3 | 12 |
| 21 | 4 | 60 |
| 22 | 5 | 25 |
| 23 | 8 | 40 |
| Vgl.* | - | 75 |

| | | |
|---|---|---|
| * Standard-Positivdruckplatte ^{(R)}OZASOL P61 | | |

Eine Platte entsprechend Beispiel 20 wurde in eine Offsetdruckmaschine eingespannt und lieferte mehr als 100.000 Drucke in guter Qualität.

### Beispiel 24

Eine Platte aus elektrochemisch aufgerauhtem und anodisiertem Aluminium wurde mit einer Lösung aus
- 27,00 Gt: eines Novolakharzes (^{(R)}Alnovol PN 429/430),
- 8,00 Gt: eines polymeren Orthoesters, hergestellt durch Umsetzung von Orthoameisensäuretrimethylester und 2-Ethyl-2-(4-hydroxy-butoxymethyl)-propan-1,3-diol,
- 0,70 Gt: Diazonium-1,1,2,3,3,3-hexafluorpropansulfonat gemäß Tabelle 1, Beispiel 3,
- 0,20 Gt: Kristallviolettchlorid und
- 675 Gt: Butanon
schleuderbeschichtet und bei 100 °C im Trockenschrank erhitzt. Nach dem Trocknen betrug die Schichtdicke 1,9 µm. Die Platte wurde unter einer 5kW-Metallhalogenidlampe im Abstand von 110 cm 15 s durch einen Halbtonstufenkeil mit 13 Stufen einer Dichteabstufung von 0,15 belichtet und in einem wäßrig-alkalischen Entwickler der Zusammensetzung gemäß der Beispiele 20 bis 23, 1:1 verdünnt mit Wasser, 30 s lang entwickelt. Man erhielt ein positives Bild der Filmvorlage, wobei die Stufe 3 des verwendeten Halbtonstufenkeils auf der Platte vollständig offen wiedergegeben wurde.

### Beispiele 21 bis 27

Eine Platte aus elektrochemisch aufgerauhtem und anodisiertem Aluminium wurde mit einer Lösung aus
- 9,00 Gt: eines Novolakharzes (^{(R)}Alnovol PN 429/430),
- 2,00 Gt: eines Veresterungsprodukts aus 1 mol 2,3,4-Trihydroxybenzophenon und 3 mol 1,2-Naphthochinon-2-diazid-5-sulfonsäurechlorid,
- 0,20 Gt: Kristallviolettchlorid und
- 175 Gt: Butanon,
zu der jeweils die in Tabelle 4 aufgeführten Gewichtsteile verschiedener Substanzen als Kontrastbildner zugesetzt wurden, schleuderbeschichtet und bei 100 °C im Trockenschrank erhitzt. Nach dem Trocknen betrug die Schichtdicke 1,9 µm. Die Platten wurden zur Hälfte mit schwarzer, lichtundurchlässiger Folie abgedeckt und unter einer 5kW-Metallhalogenidlampe im Abstand von 110 cm jeweils 100 s lang belichtet. Nach dem Ausmessen des entstandenen Bildkontrasts als Differenz der Volltondichten der unbelichteten und belichteten Bereiche wurden die Platten bei 100 °C gelagert und die Abnahme des Bildkontrasts mit der Zeit beobachtet. Tabelle 4 zeigt die überlegene thermische Stabilität des Bildkontrasts bei Verwendung der erfindungsgemäßen Diazonium-1,1,2,3,3,3-hexafluorpropansulfonate.

**Tabelle 4**

| Beispiel Nr. | Kontrastbildner | Gt | Bildkontrast | | |
|---|---|---|---|---|---|
| | | | Nullwert | 15 min | 4 h |
| 25 | 1,2-Naphthochinon-2-diazid-4-sulfonsäurechlorid | 0,3 | 0,24 | 0,03 | - |
| 26 | 2-(4-Stilbenyl-)4,6-bis-trichlormethyl-s-triazin | 0,1 | 0,25 | 0,02 | - |
| 27 | Diazonium-1,1,2,3,3,3-hexafluorpropansulfonat 3 gemäß Tabelle 1 | 0,45 | 0,29 | 0,28 | 0,25 |

### Beispiel 28

Eine 125 µm dicke, haftvermittelnd beschichtete Polyethylenterephthalatfolie wurde mit einer Lösung aus
- 67,5 Gt: Aceton,
- 17,5 Gt: Methanol,
- 4,0 Gt: n-Butanol,
- 4,0 Gt: Ethylenglykol-monomethylether,
- 7,0 Gt: Celluloseacetopropionat,
- 0,2 Gt: 5-Sulfosalicylsäure,
- 0,1 Gt: 2,4-Dihydroxy-benzoesäure,
- 1,7 Gt: Diazoniumhexafluorpropansulfonat gemäß Tabelle 1, Beispiel 9, und
- 1,0 Gt: 2,2'-Dimethyl-4,4'-dihydroxy-5,5'-di-tert.-butyl-diphenylsulfid
beschichtet und 1 min im Umlufttrockenschrank bei 90 °C getrocknet. Nach dem Trocknen betrug das Schichtgewicht 8 g/m². Der erhaltene Duplizierfilm wurde durch eine transparente Strichvorlage belichtet und in feuchtem Ammoniakgas entwickelt. Man erhielt eine positive Kopie der Filmvorlage mit orangeroten Linien auf transparentem Hintergrund.

## Patentansprüche

1. Als Photostarter in strahlungsempfindlichen Gemischen geeignete aromatische oder heteroaromatische Mono- oder Bis-diazonium-1,1,2,3,3,3-hexafluorpropansulfonate, in denen das aromatische oder heteroaromatische Kation 4 bis 20 Kohlenstoffatome enthält, wobei die Heteroatome in den heteroaromatischen Verbindungen Sauerstoff-, Schwefel- und/oder Stickstoffatome sind.

2. Benzoldiazonium-1,1,2,3,3,3-hexafluorpropansulfonate und Fluorendiazonium-1,1,2,3,3,3-hexafluorpropansulfonate, die gegebenenfalls mit Halogenatomen, Hydroxy-, (C₁-C₄)-Alkoxy-, Phenoxy-, (C₁-C₄)Alkyl-, Amino-, (C₁-C₄)Alkylamino-, Di(C₁-C₄)alkylamino-, (C₁-C₄)Hydroxyalkylamino-, Di(C₁-C₄)hydroxyalkylamino-, (C₁-C₄)Hydroxyalkyl-(C₁-C₄)alkylamino-, Pyrrolidino-, Piperidino-, Morpholino-, Phenylamino-, Nitro-, Phenyl- oder Phenylazo-, Phenylmercapto- oder Benzoylgruppen substituiert sind, wobei die Benzoylgruppen ihrerseits mit einer (C₁-C₄)Alkyl-, (C₁-C₄)Alkoxy- oder Diazoniumgruppe und die Phenylmercaptogruppen mit einer (C₁-C₄)Alkyl- oder (C₁-C₄)Alkoxygruppe substituiert sein können.

3. Dibenzofuran-x-diazonium-1,1,2,3,3,3-hexafluorpropansulfonate, Dibenzo[b,d]thiophen-x-diazonium-1,1,2,3,3,3-hexafluorpropansulfonate, 9-(C₁-C₄)Alkyl-carbazol-x-diazonium-1,1,2,3,3,3-hexafluorpropansulfonate, wobei x die Positionsziffer angibt.

4. Strahlungsempfindliches Gemisch, das
a) eine Verbindung, die unter der Einwirkung aktinischer Strahlung Säure bildet,
b¹) für ein positiv-arbeitendes Gemisch eine Verbindung, deren Löslichkeit in wäßrig-alkalischer Lösung unter der Einwirkung der Strahlung oder der Säure zunimmt, oder
b²) für ein negativ-arbeitendes Gemisch eine Verbindung, deren Löslichkeit in wäßrig-alkalischer Lösung unter der Einwirkung der Säure abnimmt, und gegebenenfalls
c) ein wasserunlösliches, in wäßrig-alkalischer Lösung dagegen lösliches oder zumindest quellbares polymeres organisches Bindemittel
enthält, dadurch gekennzeichnet, daß die Verbindung a) ein aromatisches oder heteroaromatisches Diazoniumsalz der 1,1,2,3,3,3-Hexafluorpropansulfonsäure ist.

5. Strahlungsempfindliches Gemisch nach Anspruch 4, dadurch gekennzeichnet, daß Verbindung b¹), deren Löslichkeit in wäßrig-alkalischer Lösung unter der Wirkung der Säure zunimmt, ein Polymer mit säurelabilen Gruppen ist.

6. Strahlungsempfindliches Gemisch nach Anspruch 5, dadurch gekennzeichnet, daß das Polymer mit säurelabilen Gruppen ein Polymer mit mindestens einer durch Säure spaltbaren C-O-C-Bindung ist, vorzugsweise ein Polymer, in dem aromatische Ringe mit säurespaltbaren tert.-Butoxycarbonyloxy-, tert.-Butoxycarbonyl- oder tert.-Butoxygruppen substituiert sind.

7. Strahlungsempfindliches Gemisch nach Anspruch 4, dadurch gekennzeichnet, daß die Verbindung b¹), deren Löslichkeit in wäßrig-alkalischer Lösung unter der Einwirkung der aktinischen Strahlung zunimmt, ein ortho-Chinondiazid, vorzugsweise ein ortho-Naphthochinondiazid, ist.

8. Strahlungsempfindliches Gemisch nach Anspruch 7, dadurch gekennzeichnet, daß das ortho-Naphthochinondiazid ein Ester aus 1,2-Naphthochinon-2-diazid-4- oder -5-sulfonsäure und einer Verbindung mit phenolischen Hydroxygruppen, vorzugsweise einer Verbindung mit 2 bis 6 phenolischen Hydroxygruppen, ist.

9. Strahlungsempfindliches Gemisch nach Anspruch 4, dadurch gekennzeichnet, daß die Verbindung b²) mindestens zwei säurevernetzbare Gruppen enthält, vorzugsweise Hydroxymethyl-, Alkoxymethyl- oder Oxiranylmethyl-Gruppen.

10. Strahlungsempfindliches Gemisch nach Anspruch 9, dadurch gekennzeichnet, daß die Verbindung b²) ein mindestens zwei N-Hydroxymethyl-, N-Alkoxymethyl- oder N-Acyloxymethylgruppen enthaltendes Melamin-Formaldehyd- oder Harnstoff-Formaldehyd-Kondensat ist.

11. Strahlungsempfindliches Gemisch nach einem oder mehreren der Ansprüche 4 bis 10, dadurch gekennzeichnet, daß das Bindemittel c) ein Polymer mit phenolischen Hydroxygruppen oder mit Carboxygruppen ist, bevorzugt ein Novolak oder ein Polyhydroxystyrol.

12. Aufzeichnungsmaterial mit einem Träger und einer strahlungsempfindlichen Schicht, dadurch gekennzeichnet, daß die Schicht aus einem strahlungsempfindlichen Gemisch gemäß einem oder mehreren der Ansprüche 4 bis 11 besteht.

## Claims

1. Aromatic and heteroaromatic mono- or bis-diazonium 1,1,2,3,3,3-hexafluoropropanesulphonates suitable as photostarters in photosensitive mixtures, wherein the aromatic or heteroaromatic cation contains 4 to 20 carbon atoms, the heteroatoms in the heteroaromatic compounds being oxygen, sulphur and/or nitrogen atoms.

2. Benzenediazonium 1,1,2,3,3,3-hexafluoropropanesulphonates and fluorenediazonium 1,1,2,3,3,3-hexafluoropropanesulphonates which may be substituted with halogen atoms, hydroxy, (C₁-C₄)alkoxy, phenoxy, (C₁-C₄)alkyl, amino, (C₁-C₄)alkylamino, di(C₁-C₄)alkylamino, (C₁-C₄)hydroxyalkylamino, di(C₁-C₄)hydroxyalkylamino, (C₁-C₄) hydroxyalkyl-(C₁-C₄)alkylamino, pyrrolidino, piperidino, morpholino, phenylamino, nitro, phenyl or phenylazo groups, phenylmercapto groups or benzoyl groups, the benzoyl groups optionally being substituted with a (C₁-C₄)alkyl, (C₁-C₄) alkoxy or diazonium group and the phenylmercapto groups optionally being substituted with a (C₁-C₄)alkyl or (C₁-C₄)alkoxy group.

3. Dibenzofuran-x-diazonium 1,1,2,3,3,3-hexafluoropropanesulphonates, dibenzo-[b,d]-thiophene-x-diazonium 1,1,2,3,3,3-hexafluoropropanesulphonates and 9-(C₁-C₄)alkylcarbazole-x-diazonium 1,1,2,3,3,3-hexafluoropropanesulphonates wherein x denotes the position number.

4. Photosensitive mixture containing :
a) a compound forming an acid upon exposure to actinic radiation,
b¹) in case of a positive-working mixture, a compound the solubility of which in an aqueous-alkaline solution increases upon exposure to the radiation or to the acid, or
b²) in case of a negative-working mixture, a compound the solubility of which in an aqueous-alkaline solution decreases upon exposure to the acid, and optionally
c) an organic polymer binder which is insoluble in water, but on the other hand is soluble or at least swellable in aqueous-alkaline solutions.
characterised in that compound a) is an aromatic or heteroaromatic diazonium salt of the 1,1,2,3,3,3-hexafluoropropanesulphonic acid.

5. Photosensitive mixture according to claim 4, characterised in that compound b¹), the solubility of which in an aqueous-alkaline solution increases upon exposure to the acid, is a polymer containing acid-labile groups.

6. Photosensitive mixture according to claim 5, characterised in that the polymer containing acid-labile groups is a polymer containing at least one acid-cleavable C-O-C bond, preferably a polymer in which the aromatic nuclei are substituted with acid-cleavable tert.-butoxycarbonyloxy, tert.-butoxycarbonyl or tert.-butoxy groups.

7. Photosensitive mixture according to claim 4, characterised in that compound b¹), the solubility of which in an aqueous-alkaline solution increases upon exposure to actinic radiation, is an ortho-quinone diazide, preferably an ortho-naphthoquinone diazide.

8. Photosensitive mixture according to claim 7, characterised in that the ortho-naphthoquinone diazide is an ester of 1,2-naphthoquinone-2-diazide-4- or -5-sulphonic acid and a compound containing phenolic hydroxyl groups, preferably a compound containing 2 to 6 phenolic hydroxyl groups.

9. Photosensitive mixture according to claim 4, characterised in that compound b²) contains at least two acid-crosslinkable groups, preferably hydroxymethyl, alkoxymethyl or oxiranylmethyl groups.

10. Photosensitive mixture according to claim 9, characterised in that compound b²) is a melamine-formaldehyde or urea-formaldehyde condensation product containing at least two N-hydroxymethyl, N-alkoxymethyl or N-acyloxymethyl groups.

11. Photosensitive mixture according to one or more of claims 4 to 10, characterised in that binder c) is a polymer containing phenolic hydroxyl groups or carboxyl groups, preferably a novolak or a polyhydroxystyrene.

12. Recording material comprising a base and a photosensitive layer, characterised in that the layer consists of a photosensitive mixture as defined in one or more of claims 4 to 11.

## Revendications

1. 1,1,2,3,3,3-hexafluoropropanesulfonates de mono- ou de bisdiazonium aromatiques ou hétéroaromatiques appropriés comme photoinitiateurs dans des mélanges sensibles au rayonnement, dans lesquels le cation aromatique ou hétéroaromatique contient de 4 à 20 atomes de carbone, les hétéroatomes dans les composés hétéroaromatiques étant des atomes d'oxygène, des atomes de soufre et/ou des atomes d'azote.

2. 1,1,2,3,3,3-hexafluoropropanesulfonates de benzènediazonium et 1,1,2,3,3,3-hexafluoropropanesulfonates de fluorènediazonium qui portent le cas échéant un ou plusieurs substituants identiques ou différents choisis parmi le groupe comprenant un atome d'halogène, un groupe hydroxyle, un groupe alcoxy en C₁-C₄, un groupe phénoxy, un groupe alkyle en C₁-C₄, un groupe amino, un groupe alkyl(en C₁-C₄)amino, un groupe dialkyl(en C₁-C₄)amino, un groupe hydroxyalkyl(en C₁-C₄)amino, un groupe dihydroxyalkyl(en C₁-C₄)amino, un groupe hydroxyalkyl(en C₁-C₄)alkyl(en C₁-C₄)amino, un groupe pyrrolidino, un groupe pipéridino, un groupe morpholino, un groupe phénylamino, un groupe nitro, un groupe phényle, un groupe phénylazoïque, un groupe phénylmercapto ou un groupe benzoyle, les groupes benzoyle pouvant porter, à titre de substituant, un groupe alkyle en C₁-C₄, un groupe alcoxy en C₁-C₄ ou un groupe diazonium et les groupes phénylmercapto pouvant porter, à titre de substituant, un groupe alkyle en C₁-C₄ ou un groupe alcoxy en C₁-C₄.

3. 1,1,2,3,3,3-hexafluoropropanesulfonates de dibenzofuranne-x-diazonium, 1,1,2,3,3,3-hexafluoropropanesulfonates de dibenzo[b,d]thiophène-x-diazonium 1,1,2,3,3,3-hexafluoropropanesulfonates de 9-alkyl(en C₁-C₄)carbazol-x-diazonium dans lesquels x représente le chiffre indiquant la position.

4. Mélange sensible au rayonnement qui contient
a) un composé qui, sous l'influence d'un rayonnement actinique, forme un acide,
b¹) pour un mélange à traitement positif, un composé dont la solubilité augmente dans une solution alcaline aqueuse sous l'influence du rayonnement ou de l'acide, ou
b²) pour un mélange à traitement négatif, un composé dont la solubilité diminue dans une solution alcaline aqueuse sous l'influence de l'acide, et le cas échéant
c) un liant organique polymère insoluble dans l'eau, mais soluble ou au moins apte à gonfler dans une solution alcaline aqueuse,
caractérisé en ce que le composé a) représente un sel de diazonium aromatique ou hétéroaromatique de l'acide 1,1,2,3,3,3-hexafluoropropanesulfonique.

5. Mélange sensible au rayonnement selon la revendication 4, caractérisé en ce que le composé b¹), dont la solubilité augmente dans une solution alcaline aqueuse sous l'influence de l'acide, représente un polymère contenant des groupes labiles en présence d'un acide.

6. Mélange sensible au rayonnement selon la revendication 5, caractérisé en ce que le polymère comprenant des groupes labiles en présence d'un acide représente un polymère contenant au moins une liaison C-O-C apte à être clivée par un acide, de préférence un polymère dans lequel des noyaux aromatiques portent, à titre de substituants, un ou plusieurs groupes tert.-butoxycarbonyloxy, tert.-butoxycarbonyle ou tert.-butoxy aptes à être éliminés par un acide.

7. Mélange sensible au rayonnement selon la revendication 4, caractérisé en ce que le composé b¹), dont la solubilité augmente dans une solution alcaline aqueuse sous l'influence du rayonnement actinique, représente un diazide d'ortho-quinone, de préférence un diazide d'ortho-naphtoquinone.

8. Mélange sensible au rayonnement selon la revendication 7, caractérisé en ce que le diazide d'ortho-naphtoquinone représente un ester de l'acide 1,2-naphtoquinone-2-diazide-4- ou 5-sulfonique et d'un composé contenant des groupes hydroxyle phénoliques, de préférence d'un composé contenant de 2 à 6 groupes hydroxyle phénoliques.

9. Mélange sensible au rayonnement selon la revendication 4, caractérisé en ce que le composé b²) contient au moins deux groupes réticulables en présence d'un acide, de préférence un groupe hydroxyméthyle, un groupe alcoxyméthyle ou un groupe oxiranylméthyle.

10. Mélange sensible au rayonnement selon la revendication 9, caractérisé en ce que le composé b²) représente un condensat de mélamine-formaldéhyde ou d'urée-formaldéhyde contenant au moins deux groupes N-hydroxyméthyle, N-alcoxyméthyle ou N-acyloxyméthyle.

11. Mélange sensible au rayonnement selon une ou plusieurs des revendications 4 à 10, caractérisé en ce que le liant c) représente un polymère contenant des groupes hydroxyle phénoliques ou des groupes carboxyle, de préférence une Novolaque ou un polyhydroxystyrène.

12. Matériau d'enregistrement comprenant un support et une couche sensible au rayonnement, caractérisé en ce que la couche est constituée par un mélange sensible au rayonnement selon une ou plusieurs des revendications 4 à 11.
